# EUROPEAN PATENT APPLICATION

(11) **EP 4 491 196 A1**
(43) Date of publication of application: **15.01.2025**
(21) Application number: 23766965.0
(22) Date of filing: 10.03.2023
(51) Int. Cl.: A61K 45/00, A61K 31/517, A61K 31/7105, A61K 31/713, A61K 39/395, A61K 48/00, A61P 9/00, A61P 43/00

(54) **COMPOSITION FOR PREVENTING OR TREATING MYOCARDITIS, COMPRISING VLA-4 INHIBITOR**

(30) Priority: 11.03.2022 JP 2022038575
(71) Applicant: OSAKA UNIVERSITY, Suita-shi Osaka 565-0871 (JP)
(72) Inventor: MATSUOKA, Ken, Suita-shi, Osaka 565-0871 (JP); TAKASHIMA, Seiji, Suita-shi, Osaka 565-0871 (JP); SEGAWA, Takatsugu, Suita-shi, Osaka 565-0871 (JP)
(74) Representative: Marks & Clerk LLP
(86) International application number: PCT/JP2023/009251
(87) International publication number: WO 2023/171789

(57) **Abstract**

An object of the present invention is to provide a novel composition for treating myocarditis and/or a novel composition for preventing the myocarditis.

A composition containing an inhibitor of VLA-4 for preventing or treating the myocarditis is provided.

## Description

### Technical Field

The present description discloses a composition for preventing or treating myocarditis, the composition for improving prognosis of patients with the myocarditis, the composition for preventing progression of the myocarditis, and the composition for treating the myocarditis accompanied by reduced cardiac function, containing an inhibitor of VLA-4.

### Background Art

Myocarditis is an inflammatory disease whose primary site is myocardium. The myocarditis accounts for about 10% of causes of sudden death, and about 30% of cases of cardiomyopathy progress to chronic myocarditis (dilated cardiomyopathy). Mortality due to the myocarditis is 22%, and among the myocarditis cases, the mortality due to fulminant myocarditis is as high as 43%. According to Non Patent Literature 1, the myocarditis is classified into lymphocytic myocarditis, giant cell myocarditis, eosinophilic myocarditis, and granulomatous myocarditis based on histological characteristics. Causes of the lymphocytic myocarditis are mostly due to viral infection with coxsackie virus, adenovirus, and the like. It is believed that the giant cell myocarditis, eosinophilic myocarditis and granulomatous myocarditis are often complications of cardiotoxic agents, drugs, autoimmunity, and systemic diseases. For the drugs, it has been reported that anthracyclines, fluorouracil, immune checkpoint inhibitors, and coronavirus vaccine may cause the myocarditis. Especially with regard to the immune checkpoint inhibitors, it has been reported that the myocarditis has developed in 0.5% of patients administered with anti-PD-1 antibodies alone, 2.4% of patients administered with anti-PD-L1 antibodies alone, and 3.3% of patients administered with anti-CTLA4 antibodies alone. In foci of the myocarditis that appear to be caused by the immune checkpoint inhibitors, findings of T-lymphocyte-predominant tissue infiltration have been noted. Furthermore, the myocarditis resulting from the administration of the immune checkpoint inhibitors has a mortality of 46% when mortality due to heart failure and mortality due to cardiogenic causes are combined (Non Patent Literature 2).

### Citation List

### Non Patent Literature

Non Patent Literature 1: Kociol. R. D. et al: Recognition and Initial Management of Fulminant Myocarditis -A Scientific Statement From the American Heart Association, Endorsed by the Heart Failure Society of America and the Myocarditis Foundation. Circulation. 2020;141
Non Patent Literature 2: Mahmood, SS: J Am Coll Cardiol 2018;71:1755-64
Non Patent Literature 3: Hokibara, S: Clin Exp Immunol 1998;114:236-244
Non Patent Literature 4: Grabmaier, U: Circulation 2012;126:A14885
Non Patent Literature 5: Ammirati, E: J Am Coll Cardiol. 2019 Jul 23;74(3):299-311

### Summary of Invention

### Technical Problem

Currently, steroid pulse therapy, immunosuppressive therapy such as cyclosporine administration, and antiviral therapy are adopted as treatment for the myocarditis. In addition, it has been reported that a VLA-4-neutralizing antibody reduces an eosinophilic infiltration in a mouse model of the eosinophilic myocarditis (Non Patent Literature 3). Furthermore, it has been reported that administration of the VLA-4-neutralizing antibody improves T cell relapse and fibrosis in a rat model of autoimmune myocarditis in the chronic state (Non Patent Literature 4). However, according to Non Patent Literature 5, prognosis has not improved over the past decade.

An object of the present invention is to provide a composition for treating the myocarditis or the composition for preventing the myocarditis.

### Solution to Problem

The present invention includes the following aspects.
Item 1. A composition containing an inhibitor of VLA-4 for preventing or treating myocarditis.
Item 2. The composition according to item 1, wherein the inhibitor of VLA-4 is an anti-VLA-4 antibody or an antigen-binding domain fragment thereof, is a VLA-4 antagonist, is at least one RNA molecule selected from a group consisting of siRNA, shRNA, miRNA, and antisense RNA targeting VLA-4 mRNA or a vector capable of expressing the RNA molecule, or is a genome editing system targeting a VLA-4 gene.
Item 3. The composition according to item 2, wherein the anti-VLA-4 antibody has a function to neutralize the VLA-4.
Item 4. The composition according to item 2, wherein the VLA-4 antagonist is carotegrast methyl.
Item 5. The composition according to any one of items 1 to 4, wherein the myocarditis is myocarditis other than eosinophilic myocarditis.
Item 6. The composition according to any one of items 1 to 5, wherein the myocarditis is myocarditis caused by an immune checkpoint inhibitor.
Item 7. The composition according to any one of items 1 to 6, wherein the myocarditis is accompanied by reduced cardiac function.
Item 8. The composition according to any one of items 1 to 7, used in combination with the composition containing an anti-Vcam-1 antibody or an antigen-binding domain fragment thereof.
Item 9. A composition containing an inhibitor of VLA-4 for preventing progression of myocarditis.
Item 10. A composition containing an inhibitor of VLA-4 for treating myocarditis accompanied by reduced cardiac function.
Item 11. A composition containing an inhibitor of VLA-4 for improving prognosis of patients suspected of having myocarditis or patients with the myocarditis.

### Advantageous Effects of Invention

According to the present invention, the myocarditis can be treated. In addition, according to the present invention, the myocarditis can also be prevented.

### Brief Description of Drawings

Fig. 1 shows macro images and low-power field and high-power field images of hematopoietic-stained tissue of a heart on Day 12, Day 19, and Day 25.
Fig. 2 shows results of population analysis of cells in cardiac muscle tissue by single-cell analysis.
Fig. 3 shows expressions of Vcam-1 in a CTL and on day 8, day 14, day 21, and day 25.
Fig. 4A shows a schedule of an adhesion experiment. Fig. 4B shows a result of Western blotting for an expression of Vcam-1. Fig. 4C shows TNFα-dependent adhesiveness between cardiac fibroblasts and monocytes.
Fig. 5A shows a schedule of an experiment. Fig. 5B shows an effect of siVcam-1 on suppressing Vcam-1 gene expression. Fig. 5C shows adhesiveness between the cardiac fibroblasts and the monocytes.
Fig. 6A shows an administration schedule of a Vcam-1-neutralizing antibody and a VLA-4-neutralizing antibody. Fig. 6B shows left ventricular fractional shortening (% fractional shortening; FS) measured by echocardiography in all mice in a CTL and neutralizing antibody groups. Fig. 6C shows boxplots of left ventricular end-diastolic diameter and left ventricular fractional shortening on day 28 in the same mice as in Fig. 6B. Fig. 6D shows Kaplan-Meier curves for the CTL and neutralizing antibody-administered groups.
Fig. 7A shows an administration schedule of the neutralizing antibody. Fig. 7B shows Kaplan-Meier curves for each group.
Fig. 8A shows %FS values of wild-type mice (WT), F1 obtained by crossing the wild-type mice with MRL-Pdcd1^{-/-} mice (hetero: MRL-Pdcd1⁻), and the MRL-Pdcd1^{-/-} mice (homo) at 3 weeks after birth. Fig. 8B shows results of dividing the homo group into a high %FS group (good cardiac function group, n=11) and a low %FS group (reduced cardiac function group, n=20). Fig. 8C shows an administration schedule of the VLA-4-neutralizing antibody.
Fig. 9 shows clinical data for the CTL, VLA-4, and normal EF groups.
Fig. 10 shows Kaplan-Meier curves for the CTL group (sign "a") and the VLA-4 group (sign "b").
Fig. 11 shows Kaplan-Meier curves for the CTL group (sign "a"), the VLA-4 group (sign "c") and the normal EF group (sign "b").
Fig. 12 shows Kaplan-Meier curves for each group when the MRL-Pdcd1^{-/-} mice were administered with an inhibitor of VLA4. In the figure, the sign "a" denotes an AJM300(+) group, and the sign "b" denotes an AJM300(-) group.
Fig. 13 shows results of heart rate and left ventricular contractile performance: % Fractional Shortening (%) for each group when the MRL-Pdcd1^{-/-} mice were administered with the inhibitor of VLA4.
Fig. 14 shows levels of soluble Vcam-1 in sera of three healthy subjects (CTL), four patients with Covid-related myocarditis (Covid related), eight patients with lymphocytic myocarditis (Lym), six patients with eosinophilic myocarditis (Eosi), and three patients with giant cell myocarditis (Giant).
Fig. 15 shows levels of the sVcam-1 in sera of the patients with lymphocytic myocarditis in acute and convalescent phases.

### Description of Embodiments

### 1. Composition for preventing or treating myocarditis

A composition for preventing or treating myocarditis according to this embodiment contains an inhibitor of VLA-4.

In the present description, the "myocarditis" is an inflammatory disease whose primary site is myocardium. The myocarditis includes acute and chronic myocarditis according to a classification based on clinical course. In addition, the acute myocarditis includes fulminant myocarditis and the myocarditis other than the fulminant myocarditis. The chronic myocarditis includes dilated cardiomyopathy with ventricular hypertrophy and the chronic myocarditis other than the dilated cardiomyopathy.

In addition, the myocarditis includes lymphocytic myocarditis, giant cell myocarditis, eosinophilic myocarditis, granulomatous myocarditis, and the like according to a histological classification. It has been reported that the lymphocytic myocarditis is caused by viral infection. It is believed that most cases are caused by the viral infection due to pathogenic virus such as enteroviruses including coxsackie virus (group A or group B), echovirus, poliovirus, and the like; hepatitis virus (type A or type C); influenza virus (type A or type B); respiratory syncytial virus; mumps virus; measles virus; dengue virus; yellow fever virus; chikungunya virus; rabies virus; HIV virus; vaccinia virus; herpes zoster virus; cytomegalovirus; herpes simplex virus; EB virus; measles virus; adenovirus; parvovirus, and the like. It has been reported that the giant cell myocarditis, the eosinophilic myocarditis, and the giant cell myocarditis are caused by bacterial infection. It has been reported that the eosinophilic myocarditis is caused by fungal infection. It is believed that the granulomatous myocarditis is often associated with infectious diseases such as rickettsial infection, spirochete infection, protozoan infection, and parasitic infection; ingestion of drugs and chemicals; allergies and autoimmune diseases; Kawasaki disease; sarcoidosis; radiation exposure; heatstroke, and others. The drugs that may cause the myocarditis may include anthracyclines, fluorouracil, immune checkpoint inhibitors, coronavirus vaccine, and the like. The immune checkpoint inhibitors may include anti-PD-1 antibody, anti-PD-L1 antibody, and anti-CTLA4 antibody. The composition according to this embodiment can be suitably used for patients with the myocarditis other than, preferably, the eosinophilic myocarditis, in particular, the patients suspected of having or diagnosed with the myocarditis caused by the immune checkpoint inhibitors.

For diagnosis of the myocarditis, in general, cardiac muscle tissue is obtained by endomyocardial biopsy to make a definitive diagnosis based on images of the cardiac muscle tissue. Thus, the patient with the myocarditis is intended to be the patient with the definitive diagnosis of the myocarditis. The patient suspected of having the myocarditis is intended to be the patient for whom the definitive diagnosis of the myocarditis has not yet been made, but for whom tests such as electrocardiogram; pulse rate; echocardiography (tomogram; evaluation of cardiac function such as left ventricular fractional shortening and left ventricular ejection fraction); measurement of CRP, AST, LDH, CK-MB, cardiac troponin (T or I), sVcam-1 in blood; chest X-ray; cardiac MRI, and the like, suggest a possibility of the myocarditis.

In this embodiment, preventing the myocarditis may include preventing an onset of the myocarditis, and preventing a recurrence of the myocarditis. Treating the myocarditis may include ameliorating a symptom of the myocarditis, curing the myocarditis, and preventing reduced cardiac function and heart failure due to myocarditis.

The degree of myocarditis can be evaluated by the tests, for example, the electrocardiogram; the pulse rate; the echocardiography (tomogram; evaluation of cardiac function such as left ventricular fractional shortening and left ventricular ejection fraction); the measurement of CRP, AST, LDH, CK-MB, cardiac troponin (T or I), sVcam-1 in blood; the chest X-ray; the cardiac MRI, and the like.

Whether the myocarditis is aggravated can be evaluated by comparing patient's current test data for a test item with patient's past test data for the same test item. For example, when determining whether the myocarditis is aggravated by using the test items of the left ventricular fractional shortening and the left ventricular ejection fraction as indices, if the patient's current test data of the left ventricular fractional shortening or the left ventricular ejection fraction is lower than the patient's past test data of these test items, it can be determined that the myocarditis is aggravated. On the other hand, if the patient's current test data of the left ventricular fractional shortening or the left ventricular ejection fraction is the same or higher than the patient's past test data of these test items, it can be determined that the myocarditis is not aggravated.

In the present description, the VLA-4, also referred to as integrin subunit alpha 4 or CD49D, is a protein that, in humans, is expressed from a gene registered in the National Center for Biotechnology Information as Gene ID: 3676. The VLA-4 is a ligand for vascular cell adhesion molecule 1 (VCAM1; CD106; INCAM-100).

The inhibitor of VLA-4 may include, for example, an anti-VLA-4 antibody or a fragment containing an antigen-binding domain thereof.

In the present invention, the "anti-VLA-4 antibody" is not limited as long as it can specifically bind to a VLA-4 protein and inhibit functional expression thereof. The antibody may be either a polyclonal antibody or a monoclonal antibody. Both the polyclonal antibody and the monoclonal antibody can be appropriately prepared by a method known to a person skilled in the art. When the antibody is the monoclonal antibody, the antibody may be a chimeric antibody, a humanized antibody or a human antibody prepared by a known method. Said antibody may also be an antibody fragment such as Fab, F(ab)₂, diabody, scFv, minibody, peptibody, mimetibody, and the like. Preferably, the anti-VLA-4 antibody is a neutralizing antibody. For example, the anti-VLA-4 antibody may include InVivoMAb anti-mouse/human VLA-4 (CD49d) (Clone No. PS/2, Cat No. BE0071, Bio X Cell) and Vedolizumab described in literature: Ferreira, E.F.B. et al. (Current Medicinal Chemistry, 2021, 28, 5884-5895), and the like.

In this embodiment, the VLA-4 antagonist may be a type that competitively inhibits the VLA-4 (competitive inhibition type) or a type that noncompetitively inhibits the VLA-4 (noncompetitive inhibition type). The VLA-4 antagonist is preferably the noncompetitive inhibition type. The VLA-4 antagonist may be a compound or peptide.

The VLA-4 antagonist may include, for example, carotegrast methyl (literature: WO2016/051828A1); SB683699/Firategrast, R411/valategrast, CT7758/CDP323, DS-70, and TBC3486 described in literature: Ferreira, E.F.B. et al (Current Medicinal Chemistry, 2021, 28, 5884-5895); PN-943 and MORF-057 described in literature: Slack, RJ (Nature Reviews Drug Discovery volume 21, pages 60-78 (2022)); BIO1211 and BIO5192 described in literature: Baiula, M et al (Front Chem. 2019 Jul 9;7:489); ER464195-01 described in literature: Ohkuro, M et al (Nat Commun. 2018 May 17;9(1): 1982ER464195-01); E6007 which is an analog of the ER464195-01. The PN-943 is a peptide.

Furthermore, the VLA-4 antagonist may include, for example, the following compounds described in the literature: Baiula, M et al (Front Chem. 2019 Jul 9; 7: 489).

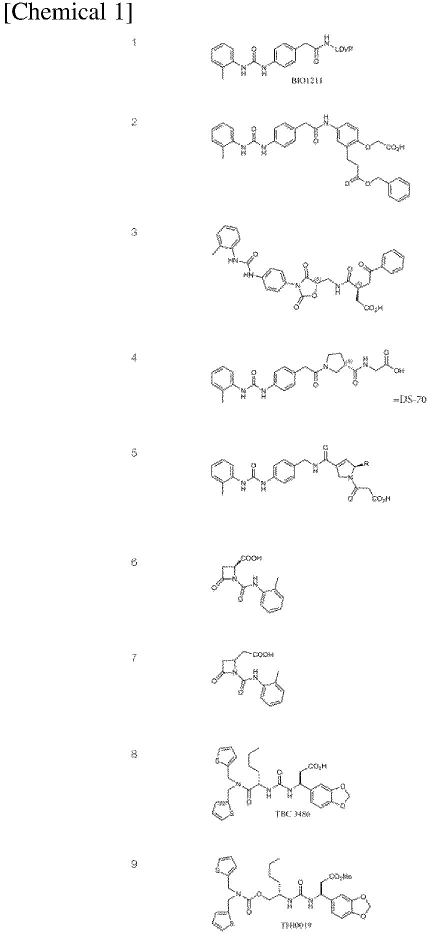

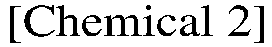

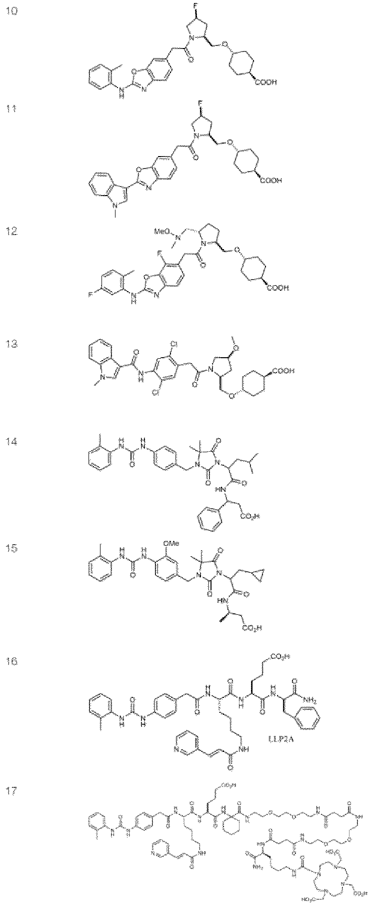

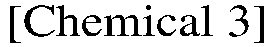

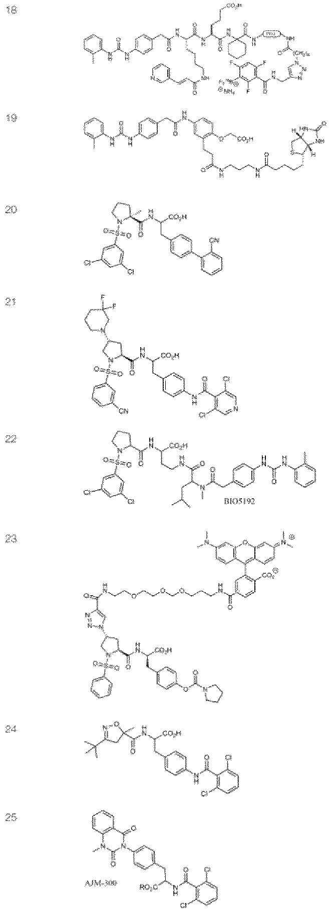

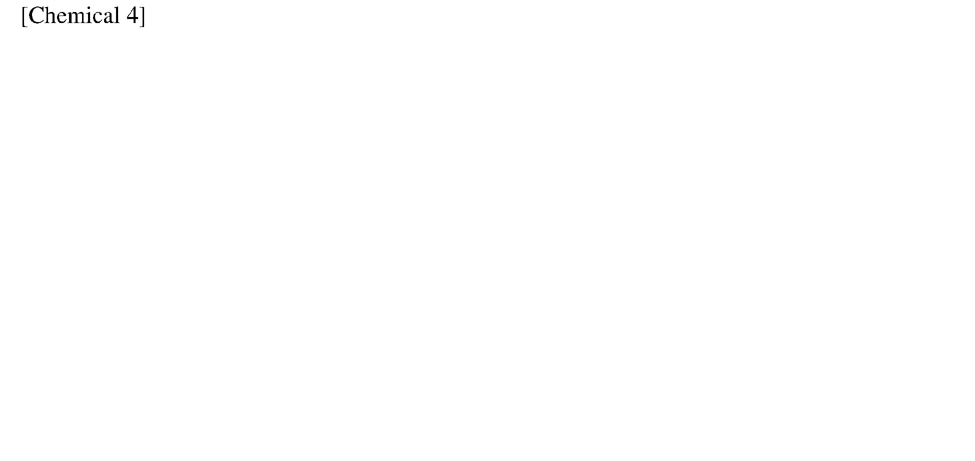

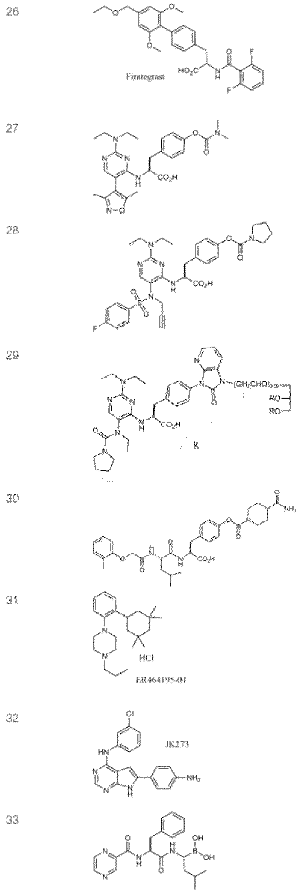

The inhibitor of VLA-4 may include an RNA molecule targeting VLA-4 mRNA. Said RNA molecule is not limited as long as it can target the VLA-4 mRNA and silence an expression of the VLA-4 mRNA. Said RNA molecule may include the RNA molecule that has an action to degrade a target mRNA, such as at least one selected from the group consisting of RNA molecules such as siRNA, shRNA, miRNA, and antisense RNA, and/or the RNA molecule that represses a translation of the target mRNA. Sequences of these RNA molecules may be designed appropriately by a person skilled in the art using a known method based on information on a nucleotide sequence of above-mentioned gene that is the target. In addition, the RNA molecule may be prepared based on a known method, or commercially available RNA molecule may be used. For example, the antisense RNA for the VLA-4 may include ATL1102 described in literature: Limmroth, V. et al (Neurology 83 November 11, 2014). The RNA molecule or a vector capable of expressing the RNA molecule may be included.

The vector capable of expressing the RNA molecule targeting the VLA-4 mRNA is not particularly limited as long as it can express the RNA molecule that silences the expression of the VLA-4 protein in the body or cells of an individual. The vector may include, for example, a hairpin RNA expression vector and the like. The hairpin RNA expression vector contains at least a sense DNA nucleotide sequence that has the same sequence as the sense strand of the target mRNA (except that an uracil in the mRNA is replaced by a thymine) downstream of a promoter nucleotide sequence suitable for expression of small RNA such as, for example, U6 promoter; a loop nucleotide sequence that forms a loop structure after transcription; an antisense DNA nucleotide sequence that can be complementarily bound in whole or in part to said sense DNA nucleotide sequence; and a terminator sequence. Examples of the vector may include a plasmid vectors, adeno-associated virus (AAV) vector, adenovirus vector, retroviral vector, lentiviral vector, and the like.

The inhibitor of VLA-4 may include a genome editing system targeting a VLA-4 gene. The genome editing system targeting the VLA-4 gene is not limited as long as it is capable of causing recombination within the VLA-4 gene in the body of the individual. The system may include, for example, CompoZr Zinc Finger Nuclease (ZFN) system, TAL effector nuclease (TALEN) system, Clustered regularly interspaced short palindromic repeats/CRISPR associated protein 9 (CRISPR/Cas9) system, and the like. The CRISPR/Cas9 system is preferred. A vector-based CRISPR/Cas9 system is preferred. In the vector-based CRISPR/Cas9 system, nucleic acids encoding the CRISPR and nucleic acids encoding the Cas9 may be on different vectors or on one vector. A promoter for allowing the CRISPR to function is not particularly limited, and the U6 promoter is preferred. A promoter for allowing the Cas9 to function is not particularly limited, and a promoter expressed in mammalian cells, such as a cytomegalovirus promoter and the like, is preferred. Preferably as the CRISPR/Cas9 system, a commercially available vector, such as pX330-U6-Chimeric_BB-CBh-hSpCas9 vector and the like, may be used.

A sequence targeting the VLA-4 gene that is to be incorporated into a CRISPR sequence (hereinafter referred to as "VLA-4 gene-targeting sequence") is not limited as long as the sequence can be incorporated into a guide RNA (gRNA, also called crRNA) and transcribed by the CRISPR/Cas9 system to recombine the VLA-4 gene. In general, it is believed that a sequence of around 20 nucleotide s in the 5' side upstream region of the nucleotide sequence "NGG" in the VLA-4 gene can be selected as the VLA-4 gene-targeting sequence. The VLA-4 gene-targeting sequence may be designed using a known design tool published in Optimized CRISPR design tool (web page of Massachusetts Institute of Technology, ZhangLab (http://crispr.mit.edu/)), E-CRISP (http://www.e-crisp.org/E-CRISP/ (German Cancer Research Center)), ZiFiT Targeter(http://zifit.partners.org/ZiFit/ (Zing Finger consortium)), Cas9 design (http://cas9.cbi.pku.edu.cn (Peking University)), CRISPRdirect (http://crispr.dbcls.jp (University of Tokyo)), CRISPR-P (http://cbi.hzau.edu.cn/crispr/ (Huazhong Agricultural University)), and the like.

The composition may be prepared by combining the inhibitor of VLA-4 with an appropriate carrier or additive. The carrier and the additive used in the preparation of the composition may include, for example, excipients, binders, disintegrants, lubricants, colorants, corrigents, flavoring agents, surfactants, and the like, which are commonly used in ordinary drugs depending on a dosage form of the composition. When the inhibitor of VLA-4 is the peptide, antibody, antibody fragment, RNA molecule, plasmid vector, and the like, a transfection reagent containing a polymer, lipid, magnetic material, etc. may be used as said carrier.

When the composition is to be administered parenterally, examples of the dosage form of the composition may include injections and infusions. In addition, when the composition is to be administered orally, the dosage form of the composition is not particularly limited, examples of the dosage form of the composition may include tablets, powders, granules, capsules (including hard and soft capsules), liquids, pills, suspensions, emulsions, and the like.

When preparing the composition as an oral solid composition, such as tablets, powders, granules, pills, capsules, and the like, the excipients such as lactose, white soft sugar, sodium chloride, glucose, urea, starch, calcium carbonate, kaolin, microcrystalline cellulose, silicic acid, methylcellulose, glycerin, sodium alginate, gum arabic, and the like; the binders such as simple syrup, glucose solution, starch solution, gelatin solution, polyvinyl alcohol, polyvinyl ether, polyvinylpyrrolidone, carboxymethylcellulose, shellac, methylcellulose, ethylcellulose, water, ethanol, potassium phosphate, and the like; the disintegrants such as dried starch, sodium alginate, powdered agar, laminaran powder, sodium bicarbonate, calcium carbonate, polyoxyethylene sorbitan fatty acid esters, sodium lauryl sulfate, stearic acid monoglyceride, starch, lactose, and the like; disintegration inhibitors such as white soft sugar, stearic acid, cocoa butter, hydrogenated oils, and the like; absorption enhancers such as sodium lauryl sulfate; moisturizers such as glycerin, starch, and the like; adsorbents such as starch, lactose, kaolin, bentonite, colloidal silicic acid, and the like; and the lubricants such as purified talc, stearates, boric acid powder, polyethylene glycol, and the like, for example, may be used as the carrier. In addition, the tablets may be tablets with conventional coatings as needed, such as sugar-coated tablets, gelatin-encapsulated tablets, enteric-coated tablets, film-coated tablets, double-coated tablets, multi-layered tablets, and the like.

When preparing the composition as the oral solid composition in the form of pills, the excipients such as glucose, lactose, starch, cacao butter, hydrogenated vegetable oil, kaolin, talc, and the like; the binders such as powdered Acacia, powdered tragacanth, gelatin, and the like; the disintegrants such as laminaran, agar, and the like, for example, may be used as the carrier.

When preparing the composition as the oral solid composition in the form of capsules, the capsules are prepared by mixing an active ingredient with the various carriers exemplified above and filling the mixture into a hard capsule, a soft capsule, or the like.

When the formulation is the liquid, it may be an aqueous or oily suspension, solution, syrup, or elixir, and is prepared according to a conventional method with a conventional additive.

When preparing the composition in the form of injections, diluents such as water, ethyl alcohol, macrogol, propylene glycol, ethoxylated isostearyl alcohol, polyoxylated isostearyl alcohol, polyoxyethylene sorbitan fatty acid esters, and the like; pH adjusting agents such as sodium citrate, sodium acetate, sodium phosphate, and the like; buffering agents such as dipotassium phosphate, trisodium phosphate, sodium hydrogen phosphate, sodium citrate, and the like; stabilizing agents such as sodium pyrosulfite, EDTA, thioglycolic acid, thiolactic acid, and the like, for example, may be used as the carrier, and saccharides such as mannitol, inositol, maltose, sucrose, lactose, and the like, for example, may be used as a molding agent when lyophilized. In this case, the glucose or the glycerin in a quantity sufficient to prepare an isotonic solution may be contained in the pharmaceutical composition, and conventional dissolution aids, soothing agent, local anesthetics and the like may also be added. With an addition of these carriers, subcutaneous, intramuscular, and intravenous injections can be produced according to a conventional method.

When the composition is the infusion, it may be prepared by dissolving a compound to be administered in an isotonic electrolyte infusion formulation based on saline, Ringer's solution, or the like.

A dosage of the composition is not limited as long as an effect of the present invention is achieved, and may be set appropriately according to, for example, the dosage form, and age, sex, and degree of disease of the patient.

For example, when the composition contains the anti-VLA-4 antibody or the antigen-binding domain fragment thereof and the composition is administered systemically by intravenous infusion or the like, it may be administered at a dose of 0.01 to 1,000 mg/day per 1 kg of adult body weight in terms of an amount of the protein of the anti-VLA-4 antibody or the antigen-binding domain fragment thereof. For example, the administration may be performed every day, every 2 days, every 3 days, every 4 days, every 5 days, every week, every 2 weeks, every 3 weeks, or every 4 weeks. Term of the administration may be continued until the myocarditis ameliorates. Preferably, it may be administered for about 6 months from an acute phase. Amelioration of the myocarditis is intended to mean that improvement in cardiac function is observed by the echocardiography and/or that a level of the sVcam-1 or cardiac troponin in a blood (preferably serum) of the patient is below a reference value. The reference value is selected from, for example, the mean, maximum, third quartile, second quartile (median), first quartile, and minimum of the level of the sVcam-1 in the blood of a healthy person.

For example, when the composition contains the VLA-4 antagonist and the composition is administered systemically by intravenous infusion or the like, it may be administered at a dose of 0.01 to 1,000 mg/day per 1 kg of adult body weight in terms of an amount of the VLA-4 antagonist. When the VLA-4 antagonist is the carotegrast methyl, the composition may be administered orally, and may be administered at a dose of 0.01 to 1,500 mg/day per kg of adult body weight in terms of an amount of the carotegrast methyl. For example, the administration may be performed every day, every 2 days, every 3 days, every 4 days, every 5 days, every 1 week, every 2 weeks, every 3 weeks, or every 4 weeks. Term of the administration may be continued until the myocarditis ameliorates. Preferably, it may be administered for about 6 months from the acute phase. Definitions of the amelioration of the myocarditis and the reference value are as described above.

At least one RNA molecule selected from the group consisting of siRNA, shRNA, miRNA, and antisense RNA targeting the VLA-4 mRNA, or a vector capable of expressing the RNA molecule, may be administered at a dose of about 0.1 to 1,000 mg/day per 1 kg of adult body weight when administered systemically. The vector may be linearized as needed.

When the at least one RNA molecule selected from the group consisting of siRNA, shRNA, miRNA, and antisense RNA targeting the VLA-4 mRNA, or the vector capable of expressing the RNA molecule is locally administered, the at least one RNA molecule selected from the group consisting of siRNA, shRNA, miRNA, and antisense RNA targeting the VLA-4 mRNA, or the vector capable of expressing the RNA molecule may be injected into a target tissue using a syringe or catheter. In this case, a nucleic acid delivery reagent such as a liposome may be used in combination. For the local administration, the dose may be 0.01 to 100 mg/day per 1 cm² of the target tissue.

The at least one RNA molecule selected from the group consisting of siRNA, shRNA, miRNA, and antisense RNA targeting the VLA-4 mRNA, or the vector capable of expressing the RNA molecule may be administered single or multiple times, whether for systemic or local administration. In the case of multiple administrations, the administration may be repeated every 2 days, every 4 days, or every week. In the case of multiple administrations, they may be administered 2, 5, 10, 15, 20 or 24 times. Preferably, they may be administered for about 6 months from the acute phase. Definitions of the amelioration of the myocarditis and the reference value are as described above.

When the genome editing system targeting the VLA-4 gene is administered to the individual, it may be administered systemically or locally. For the systemic administration, the intravenous injection is preferred. If the nucleic acid contained in the genome editing system is a DNA-based system, a vector derived from lentivirus, adenovirus, AAV, and the like, that can be expressed in the individual, may be used as the vector.

When the genome editing system targeting the VLA-4 gene is administered systemically, it may be administered at 10¹⁰ to 10¹⁸ vg/day per 1 kg of adult body weight. The genome editing system may be administered single or multiple times, whether for systemic or local administration. In the case of multiple administrations, the administration may be repeated every 2 days, every 4 days, or every week. In the case of multiple administrations, it may be administered 2, 5, 10, 15, 20 or 24 times. Preferably, it may be administered for about 6 months from the acute phase. Definitions of the amelioration of the myocarditis and the reference value are as described above. It may be administered to the individual in combination with the nucleic acid delivery reagent such as the liposome. If the nucleic acid contained in the genome editing system is an RNA-based system, it may be administered in combination with the liposome. In addition, the vector is preferably linearized as needed.

### 2. Composition for treating myocarditis accompanied by reduced cardiac function

The compositions described in the above section 1 may also be used as the composition for treating myocarditis accompanied by reduced cardiac function. Therefore, the description of the compositions in the above section 1 is incorporated herein. In addition, the description for the myocarditis in the above section 1 is also incorporated herein.

The cardiac function can be evaluated by, for example, the echocardiography (tomogram; evaluation of cardiac function such as left ventricular fractional shortening and left ventricular ejection fraction) and the like. The reference value of each test item is compared with a measurement data of each test item of the patient, and if the value of the measurement data is lower than the reference value, it can be determined that the cardiac function of the patient is reduced.

### 3. Composition for improving prognosis of patient suspected of having myocarditis or patient with the myocarditis

The compositions described in the above section 1 may also be used as the composition for improving prognosis of patients suspected of having the myocarditis or patients with the myocarditis. Therefore, the description of the compositions in the above section 1 is incorporated herein. In addition, the description for the myocarditis in the above section 1 is also incorporated herein.

In this embodiment, the prognosis is intended to mean survival time, and the improvement of the prognosis is intended to mean prolongation of the survival time. The prolongation of the survival time is intended to mean, for example, that the survival time is longer than a reference value calculated from a survival time of a group of patients who have not administered with the composition according to this embodiment. The reference value may include, for example, the median, first quartile, third quartile, mean, and the like of the survival time of the group of patients.

### 4. Composition containing anti-Vcam-1 antibody or antigen binding domain fragment thereof to be used in combination with each composition

The compositions described in the above sections 1 to 3 may be used in combination with the composition containing an anti-Vcam-1 antibody or an antigen-binding domain fragment thereof.

The anti-Vcam-1 antibody is not limited as long as it can bind specifically to a Vcam-1 protein and inhibit the functional expression of the Vcam-1 protein. The anti-Vcam-1 antibody may be either the polyclonal antibody or the monoclonal antibody. Both the polyclonal antibody and the monoclonal antibody may be appropriately prepared by a method known to a person skilled in the art. When the anti-Vcam-1 antibody is the monoclonal antibody, it may be a chimeric antibody, humanized antibody, or human antibody prepared by a known method. In addition, the anti-Vcam-1 antibody may be an antibody fragment such as Fab, F(ab)₂, diabody, scFv, minibody, peptibody, mimetybody, and the like. Preferably, the anti-Vcam-1 antibody is the neutralizing antibody. For example, the anti-Vcam-1 antibody may include InVivoMAb anti-mouse CD106 (VCAM-1) (Clone No. M/K-2.7, Cat No. BE0027, Bio X Cell).

The composition may be prepared by combining the anti-Vcam-1 antibody or the antigen-binding domain fragment thereof with the appropriate carrier or additive. The description of the carrier and additive used in the preparation of the composition in the above section 1 is incorporated herein.

When the composition containing the anti-Vcam-1 antibody or the antigen-binding domain fragment thereof is administered systemically by intravenous infusion or the like, it may be administered at a dose of 0.01 to 1,000 mg/day per 1 kg of adult body weight in terms of an amount of the protein of the anti-Vcam-1 antibody or the antigen-binding domain fragment thereof. For example, the administration may be performed every day, every 2 days, every 3 days, every 4 days, every 5 days, every 1 week, every 2 weeks, every 3 weeks, or every 4 weeks. Term of the administration may be continued until the myocarditis ameliorates.

For timing of combining the compositions described in the above sections 1 to 3 with the composition containing the anti-Vcam-1 antibody or the antigen-binding domain fragment thereof, the compositions described in the above sections 1 to 3 may be administered followed by administration of the composition containing the anti-Vcam-1 antibody or the antigen-binding domain fragment thereof, or the compositions described in the above sections 1 to 3 and the composition containing the anti-Vcam-1 antibody or the antigen-binding domain fragment thereof may be administered at the same time. Here, it is preferred to avoid administering the composition containing the anti-Vcam-1 antibody or the antigen-binding domain fragment thereof prior to administering the compositions described in the above sections 1 to 3. This is because the administration of the anti-Vcam-1 antibody or the antigen-binding domain fragment thereof may not improve the prognosis of the patient.

### Examples

Hereinafter, the present invention will be described in more detail with reference to examples. However, the present invention shall not be construed as limited to the examples.

In addition, all animal experiments in the examples were conducted according to Guide for the Care and Use of Laboratory Animals and were approved by the Animal Experiments Committee of Osaka University.

### I. Single-cell analysis using rat model of autoimmune myocarditis

### (i) rat model of autoimmune myocarditis

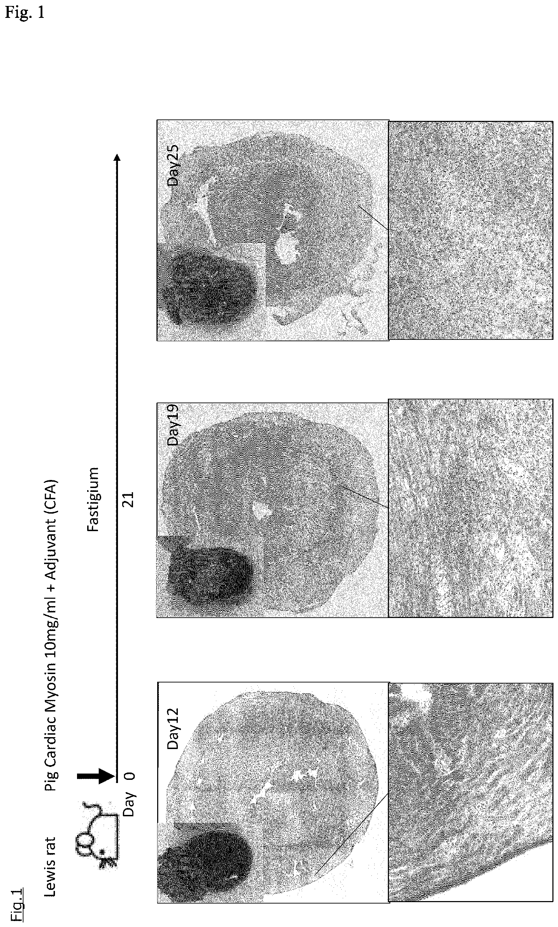

A rat model of autoimmune myocarditis was prepared by administering 10 mg/ml of Pig Cardiac Myosin (purified in-house from pig heart) and Adjuvant (CFA 10 mg/ml; Chondrex, inc., 7002) to Lewis rats (male, 8 weeks old). A day of administration of the Pig Cardiac Myosin was set as Day 0. In this model, fastigium of the myocarditis occurred on Day 21. Fig. 1 shows macro images and low-power field and high-power field images of hematopoietic-stained tissue of a heart on Day 12, Day 19, and Day 25. On Day 12, infiltration of inflammatory cells into cardiac muscle tissue began, and, on Day 19, more intense infiltration of the inflammatory cells was observed. On Day 25, destruction of the cardiac muscle tissue due to inflammation was observed. No infiltration of eosinophils was observed in any disease stage.

### (ii) Population analysis of cells in cardiac muscle tissue by single-cell analysis

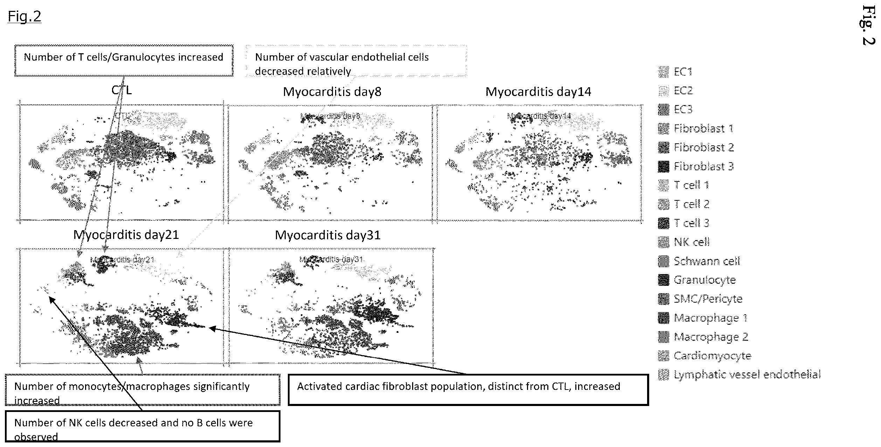

Single cell analysis was performed by dispersing cells from a rat heart with an enzyme (Liberase TM (Roche, 5401119001/Liberase DH (Roche, 5401054001)) and analyzing them with Chromium (10x Genomics). Fig. 2 shows a result. In the figure, "CTL" denotes a control without being administered with an antigen, and "Myocarditis day ..." denotes the number of days elapsed since the administration of the Pig Cardiac Myosin. On Day 21, the number of T cells and neutrophils increased, while the number of vascular endothelial cells decreased relatively. In addition, the number of monocytes and macrophages was also significantly increased, while the number of NK cells decreased and no B cells were observed. Also, no eosinophils were detected. Furthermore, as distinct from the CTL, an activated cardiac fibroblast population increased.

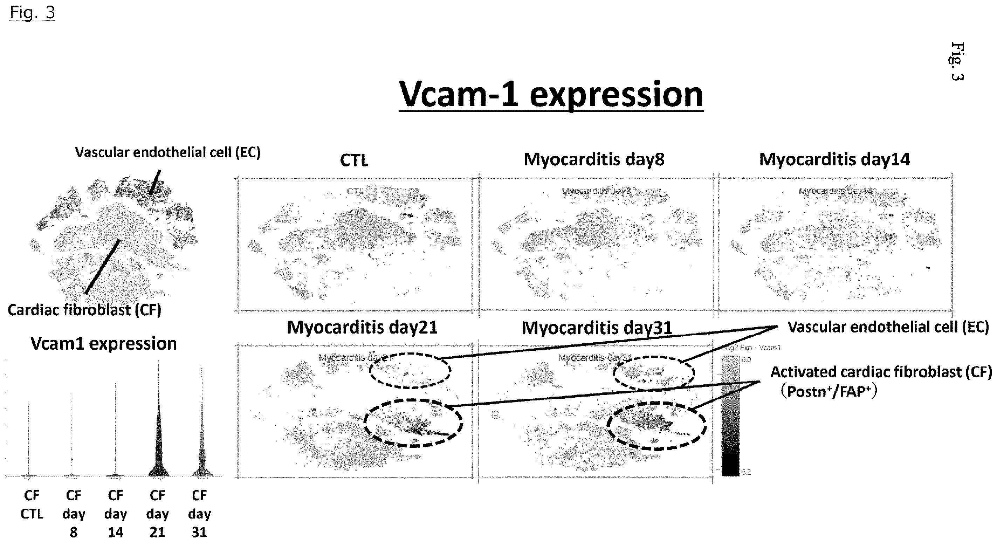

Fig. 3 shows expressions of Vcam-1 in the CTL and on day 8, day 14, day 21, and day 25. The Vcam-1 was expressed in the vascular endothelial cells, and was also highly expressed in the activated cardiac fibroblasts (Postn⁺/FAP⁺).

### II. Adhesion experiment of monocytes with cardiac fibroblasts in vitro

This experiment was conducted to determine whether the expression of the Vcam-1 is altered by stimulation of TNFα, an inflammatory cytokine, and whether the adhesiveness between the cardiac fibroblasts and the monocytes is altered by the stimulation of the TNFα.

### (i) Culture and adhesion conditions

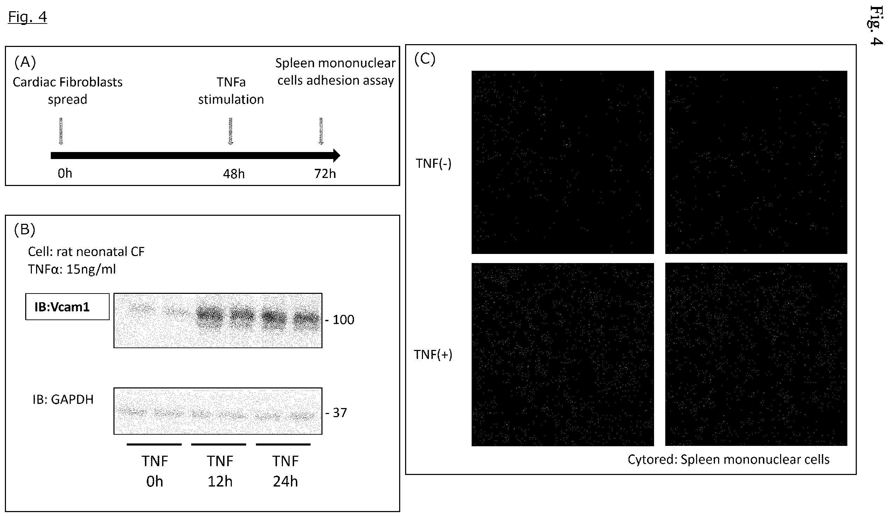

Fig. 4A shows a schedule of an adhesion experiment.

For the cardiac fibroblasts, fibroblasts isolated after cell dispersion of a heart harvested from a neonatal rat with Collagenase type 2 (Worthington Biochemical Corporation, LS004176) were cultured in a 96-well plate. For splenic monocytes, a spleen was harvested from a wild-type mouse and homogenized, and then the monocyte fraction was collected by specific gravity separation using Optiprep (Serumwerk Bernburg, 1893). For the adhesion experiment, the cardiac fibroblasts were cultured in the 96-well plate for 48 hours, and then medium was replaced with TNFα +/- and cultured for another 24 hours. The splenic monocytes subjected to fluorescent staining (Hoechst 33342 (DOJINDO LABORATORIES, 341-07901) or Cytored (DOJINDO LABORATORIES, 342-08531)) were added and incubated at 37°C for 30 min. After washing 6 times with PBS(-) to remove non-adherent monocytes, and then adherent monocytes were counted on In Cell Analyzer 6000 (GE Healthcare Life Sciences).

The TNFα (Peprotech, 300-01A) was added to the wells at a final concentration of 5 to 15 ng/ml.

Induction of the Vcam-1 by the TNFα was determined by Western blotting. Anti-Vcam1 antibody EPR5047 (Abcam, ab134047) was used as a primary antibody for the Western blotting. An HRP-conjugated anti-Rabbit antibody was used for detection, and Chemi-Lumi One L (Nacalai tesque, 07880-70) was used as a luminescence reagent. The Western blotting for GAPDH was performed as an internal control.

### (ii) Results

Fig. 4B shows results of the Western blotting. The Western blotting was performed in duplicates for each sample. The Vcam-1 was slightly expressed even at 0 hour after the addition of the TNFα. However, the expression of the Vcam-1 increased more at 12 hours and 24 hours after the addition of the TNFα. In addition, Fig. 4C is an image captured by the In Cell Analyzer 6000. As shown in Fig. 4C, it was shown that the adhesion between the cardiac fibroblasts and the monocytes was strengthened by the stimulation with the TNFα.

### III. Adhesion experiment between cardiac fibroblasts and monocytes using siRNA in vitro

To confirm direct adhesion of the Vcam-1 to the cardiac fibroblasts and the monocytes, the cardiac fibroblasts in which the V-cam-1 was knocked down using siRNA were used to determine the adhesiveness between the cardiac fibroblasts and the monocytes.

### (i) Experimental conditions

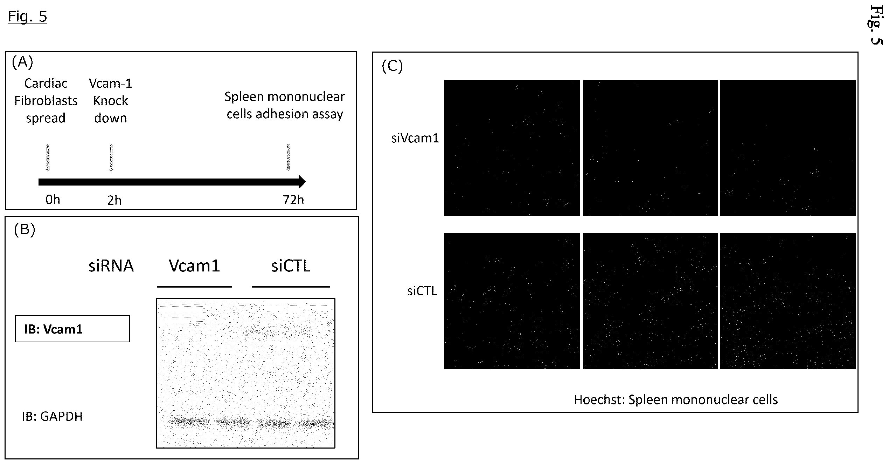

Fig. 5A shows a schedule of this experiment.

The culture of the cardiac fibroblasts and the adhesion experiment were performed as described in the above section II (i).

A Vcam-1 siRNA (Silencer^{™} Select Pre-Designed siRNA, siRNA ID s129593, Thermo; Also referred to as siVcam-1) was transfected into the cardiac fibroblasts at 2 hours after plating in wells using Lipofectamine^{™} RNAiMAX Transfection Reagent (Thermo, 13778075) at a final concentration of 30nM. The adhesion experiment with the monocytes was performed 72 hours after plating the cardiac fibroblasts into the wells. Silencer^{™} Select Negative Control No.1 siRNA (Thermo, 4390843) was used as a control for the siRNA. The Silencer^{™} Select Negative Control No.1 siRNA was transfected at the same amount of the siVcam-1. In addition, the TNFα was added at 5 ng/ml at 46 hours after the transfection.

The Western blotting for the Vcam-1 was performed as described in the above section II (i).

For detection of the monocytes, the adherent monocytes were counted on the In Cell Analyzer 6000 (GE Healthcare Life Sciences).

### (ii) Results

As shown in Fig. 5B, a signal of the Vcam-1 was detected in the cardiac fibroblasts transfected with the siCTL, whereas the signal of the Vcam-1 was not detected in the cardiac fibroblasts transfected with the siVcam-1. This indicates that the siVcam-1 suppresses the expression of the Vcam-1 in the cardiac fibroblasts. This experimental system was used to determine the adhesiveness between the cardiac fibroblasts and the monocytes. Fig. 5C shows results. The adhesion of the monocytes was reduced in the cells transfected with the siVcam-1. On the other hand, the adhesion of the monocytes was observed in the cells transfected with the siCTL. This indicates that the adhesion between the cardiac fibroblasts and the monocytes is mediated by the Vcam-1.

### IV. Verification of effect of combination of Vcam-1-neutralizing antibody and VLA-4-neutralizing antibody in improving prognosis of myocarditis

According to literature: Int Immunol. 2010 Jun; 22(6): 443-52, MRL-Pdcd1^{-/-}, which is obtained by crossing MRL-Fas^{lpr/lpr}, a spontaneous systemic lupus erythematosus model mouse, with C57BL/6-Pdcd1^{-/-}, a PD-1 knockout mouse, is a spontaneous lymphocytic myocarditis model in which 96% of the mice develop myocarditis at 4 to 8 weeks after birth. In addition, survival rate of the MRL-Pdcd1^{-/-} mice at 10 weeks after birth is about 30%, which is significantly lower than that of wild-type mice. This MRL-Pdcd1^{-/-} mouse was used in the following experiments on the myocarditis.

### (i) Combined administration of Vcam-1-neutralizing antibody and VLA-4-neutralizing antibody

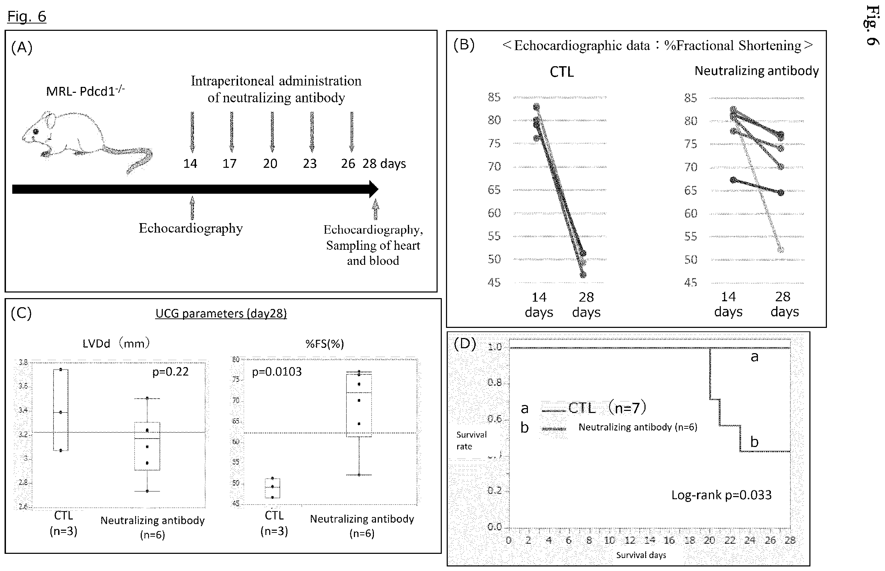

Thirteen MRL-Pdcd1^{-/-} mice were divided into two groups: control (CTL: n=7) and a combined administration of a Vcam-1-neutralizing antibody and a VLA-4-neutralizing antibody (neutralizing antibody-administered groups: n=7). From day 14 after birth, the CTL group was administered with saline intraperitoneally, and the neutralizing antibody-administered groups was administered with the Vcam-1-neutralizing antibody and the VLA-4-neutralizing antibody. InVivoMAb anti-mouse CD106 (VCAM-1) (clone No. M/K-2.7, Cat No. BE0027, Bio X Cell) was used as the Vcam-1-neutralizing antibody. InVivoMAb anti-mouse/human VLA-4 (CD49d) (clone No. PS/2, Cat No. BE0071, Bio X Cell) was used as the VLA-4-neutralizing antibody. Each dose was 10 µg/g. For an administration schedule, as shown in Fig. 6A, the administration was performed every 3 days from day 14 to day 26 after birth. The echocardiography was performed on day 14 and day 28 after birth. After acquisition of echocardiography data on day 28 after birth, blood and hearts were collected from all mice.

### (ii) Results

Fig. 6B shows left ventricular fractional shortening (% fractional shortening; FS) measured by the echocardiography in all mice in the CTL group and the neutralizing antibody-administered groups. In the CTL group, ventricular contractility was significantly reduced on day 28 after birth compared to that on day 14 after birth. On the other hand, the reduction of the ventricular contractility was milder in the neutralizing antibody-administered group than in the CTL group. Fig. 6C shows boxplots of left ventricular end-diastolic diameter [LVDd (mm)] and the left ventricular fractional shortening [%FS (%)] on day 28 in the same mice as in Fig. 6B. There was no significant difference in the LVDd between the CTL group and the neutralizing antibody-administered group (p=0.22). On the other hand, the %FS was significantly higher in the neutralizing antibody-administered group (p=0.0103). Fig. 6D shows Kaplan-Meier curves for the CTL group and the neutralizing antibody-administered group. In the figure, sign "a" denotes the CTL group and sign "b" denotes the neutralizing antibody-administered groups. In an examination up to day 28 after birth, the number of mice in the CTL group that died increased from day 20 after birth. On the other hand, no mice in the neutralizing antibody-administered group died. A significant difference between the two groups was shown by a Log-rank test (p=0.033). This indicates that the combined administration of the V cam-1-neutralizing antibody and the VLA-4-neutralizing antibody ameliorates mortality due to the myocarditis.

### V. Effect of VLA-4-neutralizing antibody

The following examination was performed to determine whether the Vcam-1-neutralizing antibody or the VLA-4-neutralizing antibody contribute to lowering the mortality due to the myocarditis.

### (i) Administration of antibody

Twenty-nine MRL-Pdcd1^{-/-} mice were divided into four groups: control (CTL: n=12), Vcam-1 group (n=5) to be administered with only the Vcam-1-neutralizing antibody, VLA-4 group (n=6) to be administered with the VLA-4-neutralizing antibody, and Vcam-1/VLA-4 group (n=6) to be administered with a combination of the Vcam-1-neutralizing antibody and the VLA-4 neutralizing antibody.

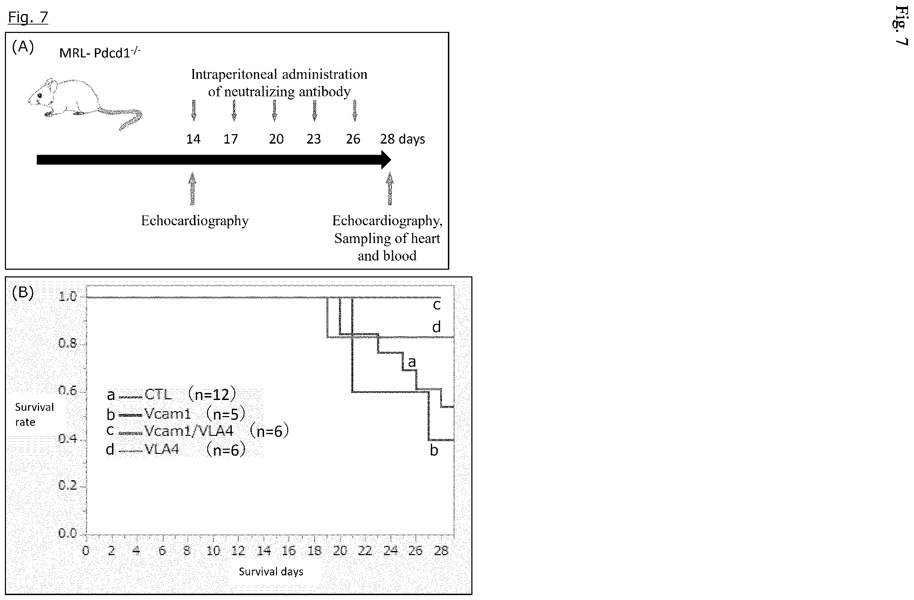

The dose of each antibody was the same as in the above section IV (i). As shown in Fig. 7A, an administration schedule of the neutralizing antibody was also the same as in the above section IV (i).

### (ii) Results

Fig. 7B shows Kaplan-Meier curves for each group. In the figure, sign "a" denotes the CTL group, sign "b" denotes the Vcam-1 group, sign "c" denotes the Vcam-1/VLA-4 group, and sign "d" denotes the VLA-4 group. In an examination up to day 28 after birth, the number of mice in the CTL group (sign "a") that died increased from day 19 after birth. The number of mice in the Vcam-1 group (sign "b") that died also increased from day 20 after birth. On the other hand, no mice in the Vcam-1/VLA-4 group (sign "c") died. In the VLA-4 group (sign "d"), one mouse died on day 18 after birth, but the remaining mice survived until day 28 after birth. This indicates that it is the VLA-4-neutralizing antibody that contributes to improving the prognosis of the myocarditis.

### VI. Effect of VLA-4-neutralizing antibody in group of mice with reduced cardiac function

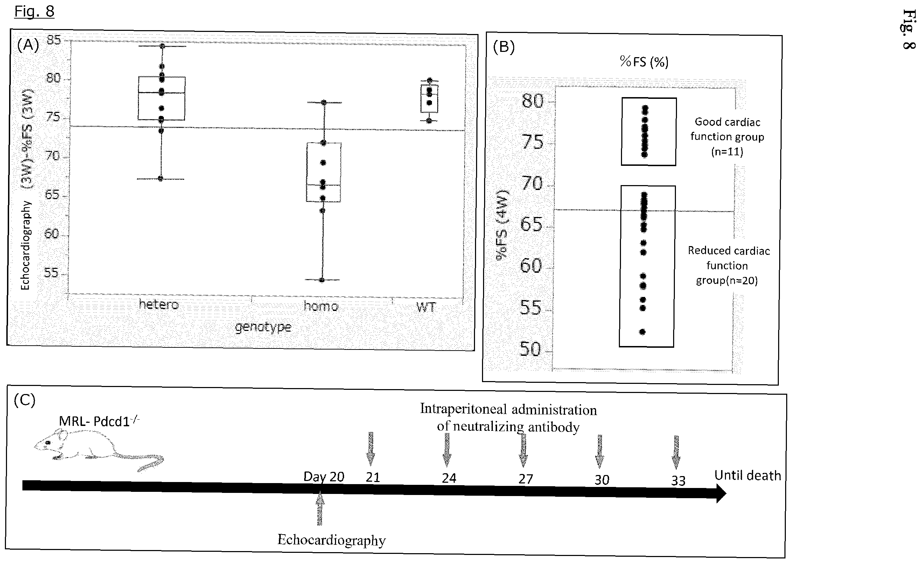

Fig. 8A shows the %FS values of the wild-type mice (WT), F1 mice (hetero: MRL-Pdcd1⁻) obtained by crossing the wild-type mice with the MRL-Pdcd1^{-/-} mice, and the MRL-Pdcd1^{-/-} mice (homo) at 3 weeks after birth. The %FS value of the WT group was more than 75%, with little difference between the first and fourth quartiles, indicating that the variation was small. On the other hand, the %FS values of the homo group and the hetero group were broadly distributed as a whole, with some individuals having high %FS values and others having low %FS values.

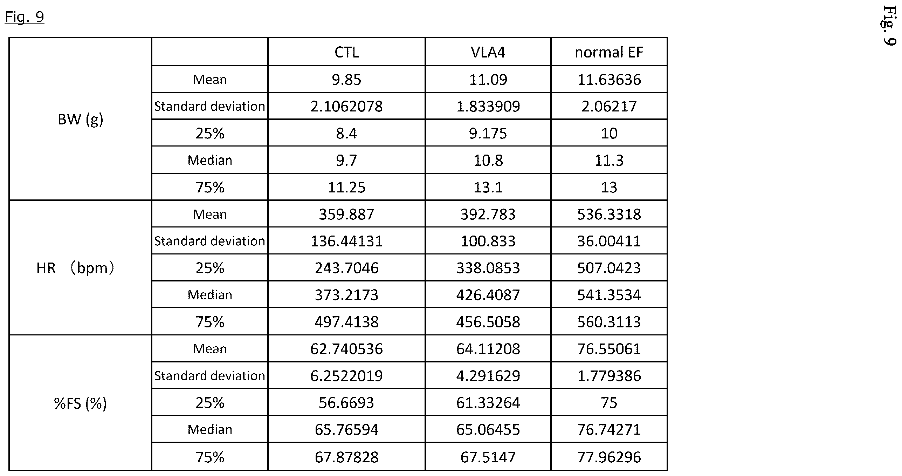

Accordingly, as shown in Fig. 8B, the homo group was divided into a high %FS group (good cardiac function group: n=11) and a low %FS group (reduced cardiac function group: n=20), and the reduced cardiac function group was further divided into a control group not to be administered with the VLA-4-neutralizing antibody (CTL group) and a VLA-4-administered group to be administered with the VLA-4-neutralizing antibody (VLA-4 group), and the effect of the VLA-4-neutralizing antibody was determined. A cutoff value to distinguish between the good cardiac function group (normal EF group) and the reduced cardiac function group was set to 70 %, and the group with more than 70 % was defined as the good cardiac function group, while the group with less than 70 % was defined as the reduced cardiac function group. Then, the VLA-4-neutralizing antibody was administered according to a schedule shown in Fig. 8C. Fig. 9 shows body weights (BW), pulse rates (HR), and the % FS values for each group. There was no significant difference in each parameter between the CTL group and the VLA-4 group.

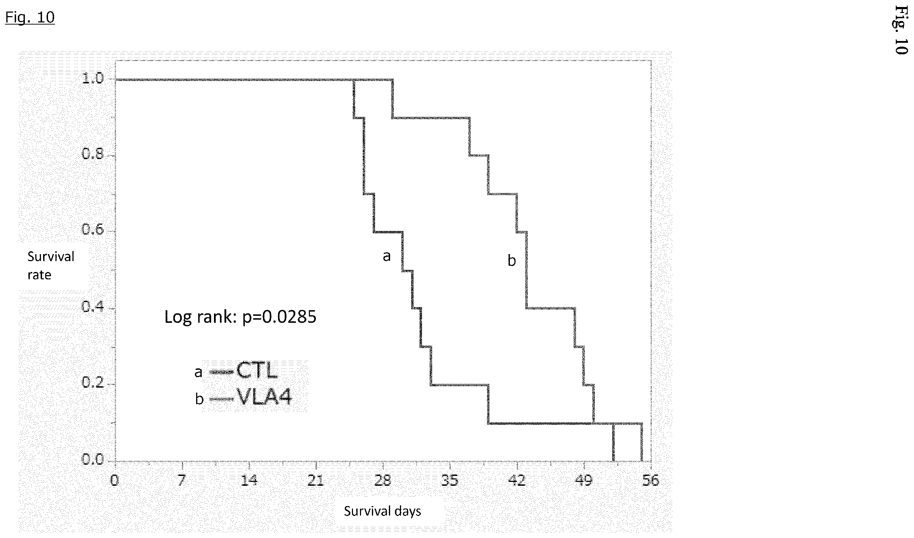

Fig. 10 shows Kaplan-Meier curves for the CTL group (sign "a") and the VLA-4 group (sign "b"). As a result of the Log-rank test, p=0.0285 was obtained, and survival rate was significantly prolonged in the VLA-4 group.

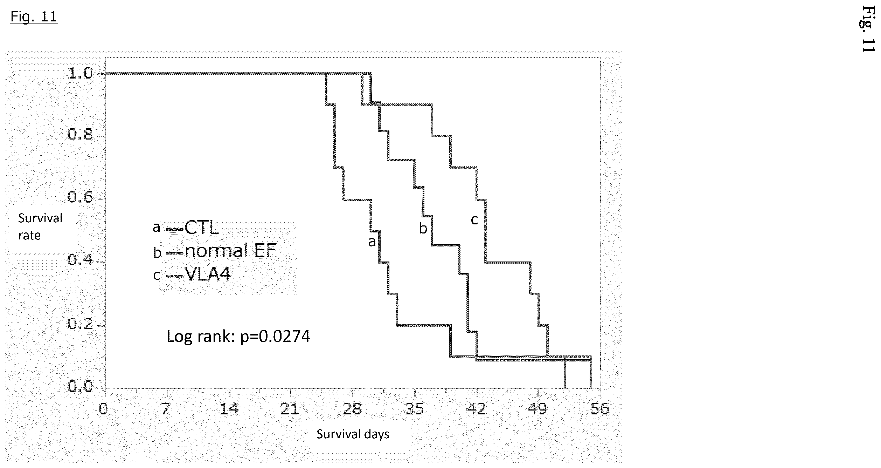

Fig. 11 shows the Kaplan-Meier curves with the addition of a survival curve for the normal EF group (sign "b"). The normal EF group, which had good cardiac function and was not administered with the VLA-4-neutralizing antibody, showed a significantly lower survival rate than the VLA-4 group (as a result of Log-rank test, p=0.0275).

This suggests that the administration of the VLA-4-neutralizing antibody to individuals with the myocarditis improves the prognosis of the myocarditis independent of cardiac function data of each individual. In addition, it is suggested that the administration of the VLA-4-neutralizing antibody to the individuals with the myocarditis is effective in preventing or treating the myocarditis.

### VII. Effect of administration of inhibitor of VLA4

The following examination was performed to determine whether administration of AJM300 (carotegrast methyl), one of the inhibitor of VLA4, contributes to lowering the mortality due to the myocarditis.

### (i) Administration of AJM300

Forty-four MRL-Pdcd1^{-/-} mice were divided into two groups: AJM300 (-) group (n=13) not to be administered with the AJM300 and AJM300 (+) group (n=12) to be administered with the AJM300.

The AJM300 group (+) was administered with a mixed diet containing 1% of AJM300 from 2 weeks of age until death.

At 28 days of age, 9 mice in the AJM300 (-) group and 10 mice in the AJM300 (+) group were evaluated for the cardiac function by the echocardiography and then euthanized to collect heart and blood samples.

### (ii) Results

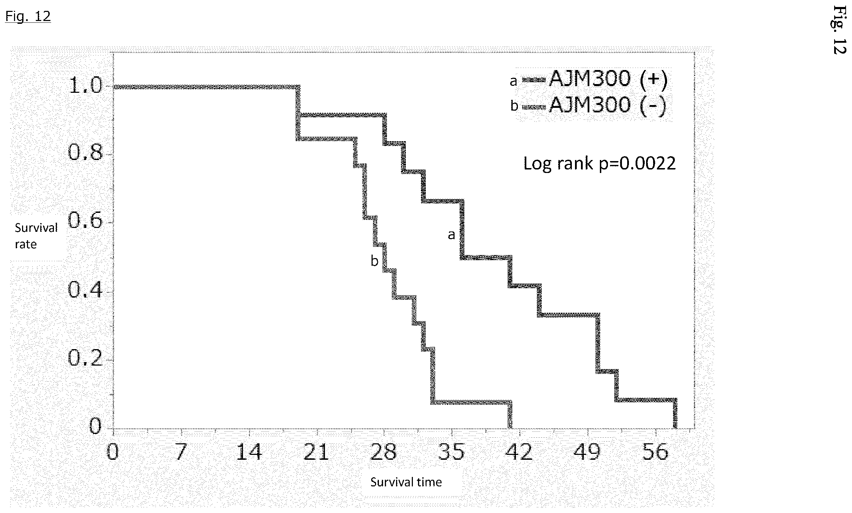

Fig. 12 shows Kaplan-Meier curves for each group. In the figure, the sign "a" denotes the AJM300(+) group, and the sign "b" denotes the AJM300(-) group. Survival time was significantly prolonged in the AJM300 (+) group compared with the AJM300 (-) group (Log-rank test, p=0.0022). This indicates that the administration of the inhibitor of VLA4 contributes to improving the prognosis of the myocarditis.

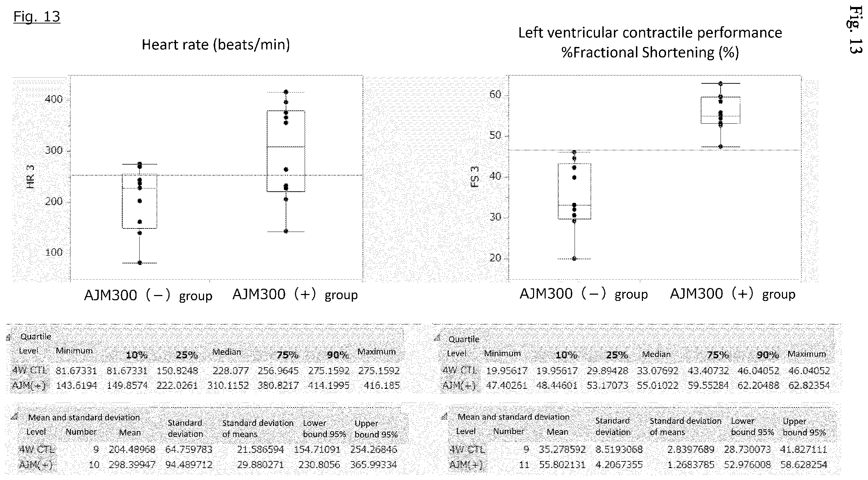

Fig. 13 shows results of the heart rate and the left ventricular contractile performance: % Fractional Shortening (%) as measured by cardiac function test by the echocardiography. The AJM300 (+) group had significantly ameliorated bradycardia compared to the AJM300 (-) group (t-test, p=0.0231). In addition, the AJM300(+) group had significantly improved left ventricular contractile performance compared to the AJM300(-) group (t-test, p=0.0231). This indicates that the administration of the inhibitor of VLA4 contributes to improving the cardiac function.

### VIII. Level of soluble Vcam-1 in serum in humans

Levels of the soluble Vcam-1 (sVcam-1) in serum were compared between healthy subjects and patients with myocarditis.

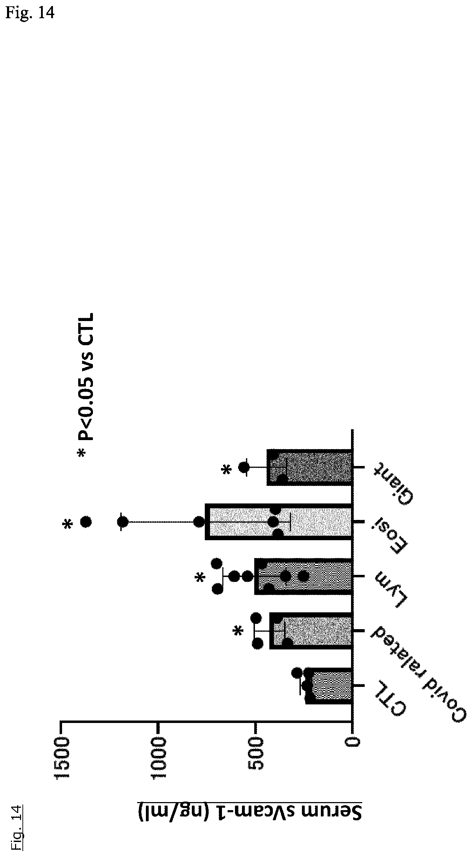

Fig. 14 shows the levels of the soluble Vcam-1 in sera of three healthy subjects (CTL), four patients with Covid-related myocarditis (Covid related), eight patients with lymphocytic myocarditis (Lym), six patients with eosinophilic myocarditis (Eosi), and three patients with giant cell myocarditis (Giant). In all types of myocarditis, including the lymphocytic myocarditis, the serum sVcam-1 levels increased compared to that in the healthy subjects. This suggests that a VLA-4/Vcam-1 pathway is involved in onset of the myocarditis.

### IX. Transition of serum sVcam-1 in myocarditis pathology

The serum sVcam-1 levels in the three patients with the lymphocytic myocarditis were compared in acute and convalescent phases.

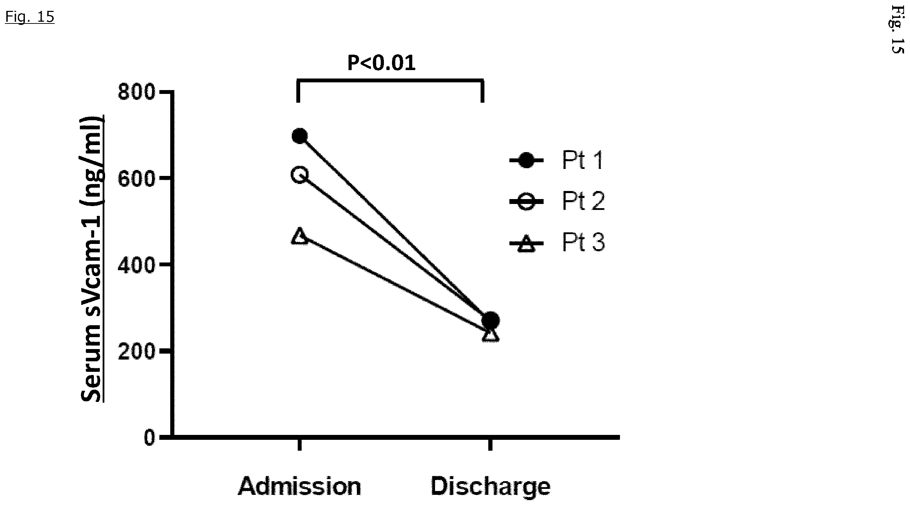

Fig. 15 shows results. The serum sVcam-1 levels of the three patients were very high in the acute phase, but improved to the level of the healthy subjects in the convalescent phase.

This suggests that the VLA-4/Vcam-1 pathway is involved in the myocarditis.

## Claims

1. A composition comprising an inhibitor of VLA-4 for preventing or treating myocarditis.

2. The composition according to claim 1, wherein the inhibitor of VLA-4
is an anti-VLA-4 antibody or an antigen-binding domain fragment thereof,
is a VLA-4 antagonist,
is at least one RNA molecule selected from a group consisting of siRNA, shRNA, miRNA, and antisense RNA targeting VLA-4 mRNA or a vector capable of expressing the RNA molecule, or
is a genome editing system targeting a VLA-4 gene.

3. The composition according to claim 2, wherein the anti-VLA-4 antibody has a function to neutralize the VLA-4.

4. The composition according to claim 2, wherein the VLA-4 antagonist is carotegrast methyl.

5. The composition according to claim 1, wherein the myocarditis is myocarditis other than eosinophilic myocarditis.

6. The composition according to claim 1, wherein the myocarditis is caused by immune checkpoint inhibitor.

7. The composition according to claim 1, wherein the myocarditis is accompanied by reduced cardiac function.

8. The composition according to claim 1, used in combination with the composition comprising an anti-Vcam-1 antibody or an antigen-binding domain fragment thereof.

9. A composition comprising an inhibitor of VLA-4 for preventing progression of myocarditis.

10. A composition comprising an inhibitor of VLA-4 for treating myocarditis accompanied by reduced cardiac function.

11. A composition comprising an inhibitor of VLA-4 for improving prognosis of patients suspected of having myocarditis or patients with the myocarditis.
